# EUROPEAN PATENT SPECIFICATION

(11) **EP 2 686 672 B1**
(45) Date of publication and mention of the grant of the patent: **03.12.2014**
(21) Application number: 12708851.6
(22) Date of filing: 15.03.2012
(51) Int. Cl.: G01N 27/30, G01N 33/00

(54) **DIAMOND BASED ELECTROCHEMICAL SENSORS**
ELEKTROCHEMISCHE SENSOREN AUF DIAMANTBASIS
CAPTEURS ÉLECTROCHIMIQUES À BASE DE DIAMANT

(30) Priority: 18.03.2011 GB 201104579; 18.03.2011 US 201161454349 P
(43) Date of publication of application: 22.01.2014
(73) Proprietor: Element Six Technologies Limited, Didcot, Oxfordshire OX11 0QR (GB)
(72) Inventor: MOLLART, Timothy Peter, Ascot Berkshire SL5 8BP (GB); SCARSBROOK, Geoffrey Alan, Ascot Berkshire SL5 8BP (GB); NEWTON, Mark Edward, Coventry West Midlands CV4 7AL (GB); MACPHERSON, Julie Victoria, Coventry West Midlands CV4 7AL (GB); UNWIN, Patrick Robert, Coventry West Midlands CV4 7AL (GB)
(74) Representative: Atkinson, Ian Anthony
(86) International application number: PCT/EP2012/054568
(87) International publication number: WO 2012/126802

(56) References cited:
- WO-A1-2005/012894
- WO-A1-2007/107844
- WO-A1-2008/090510
- US-A1- 2010 012 494

## Description

### Field of Invention

The present invention relates to a diamond based electrochemical sensor.

### Background of invention

Electrochemical sensors are well known. It has also been proposed in the prior art to provide a diamond based electrochemical sensor. Diamond can be doped with boron to form semi-conductive or fully metallic conductive material for use as an electrode. Diamond is also hard, inert, and has a very wide potential window making it a very desirable material for use as a sensing electrode for an electrochemical cell, particularly in harsh chemical, physical, and/or thermal environments which would degrade standard metal based electrochemical sensors. In addition, it is known that the surface of a boron doped diamond electrode may be functionalized to sense certain species in a solution adjacent the electrode.

One problem with using diamond in such applications is that diamond material is inherently difficult to manufacture and form into suitable geometries for sophisticated electrochemical analysis. To date, diamond electrodes utilized as sensing electrodes in an electrochemical cell have tended to be reasonably simple in construction and mostly comprise the use of a single piece of boron doped diamond configured to sense one physical parameter or chemical species at any one time. More complex arrangements have involved introducing one or more channels into a piece of boron doped diamond through which a solution can flow for performing electrochemical analysis. However, to date the present applicant is unaware of sophisticated diamond based electrochemical sensors which can perform multiple sensing functions at the same time, particularly configured for use in harsh environments. Due to the inherent difficulties involved in manufacturing and forming diamond into multi-structural components, even apparently relatively simple target structures can represent a significant technical challenge.

In terms of prior art arrangements, WO 2005012894 describes a microelectrode comprising a diamond layer formed from electrically non-conducting diamond and containing one or more pin-like projections of electrically conducting diamond extending at least partially through the layer of non-conducting diamond and presenting areas of electrically conducting diamond at a front sensing surface. In contrast, WO2007107844 describes a microelectrode array comprising a body of diamond material including alternating layers of electrically conducting and electrically non-conducting diamond material and passages extending through the body of diamond material. In use, fluid flows through the passages and the electrically conducting layers present ring-shaped electrode surfaces within the passages in the body of diamond material.

It is an aim of certain embodiments of the present invention to provide an improved diamond based electrochemical sensor capable of providing multiple sensing functions and which is constructed to be robust to harsh chemical, physical, and/or thermal environments.

### Summary of Invention

It has been found that high aspect ratio boron doped diamond electrodes have improved sensing capability when compared with other boron doped diamond electrode arrangements. That is, it has been found to be highly advantageous to provide boron doped diamond electrodes which have a high length / width ratio at a sensing surface. Furthermore, it has been found that an array of high aspect ratio boron doped diamond electrodes providing a band sensor structure can be utilized to provide multiple sensing functions. Accordingly, the present invention is directed to such sensor structures. According to the present invention there is provide a diamond based electrochemical band sensor comprising:
a diamond body; and
a plurality of boron doped diamond band electrodes disposed within the diamond body,
wherein at least a portion of each of the plurality of boron doped diamond band electrodes is doped with boron to a level suitable to achieve metallic conduction, the boron doped diamond electrodes being spaced apart by non-conductive intrinsic layers of diamond,
wherein the diamond body comprises a front sensing surface with the plurality of boron doped diamond band electrodes being exposed at said sensing surface and extending in an elongate manner across said surface, and
wherein each boron doped diamond electrode has a length / width ratio of at least 10 at the front sensing surface.

### Brief Description of the Drawings

For a better understanding of the present invention and to show how the same may be carried into effect, embodiments of the present invention will now be described by way of example only with reference to the accompanying drawings, in which:
Figure 1 illustrates a top view of a diamond based electrochemical band sensor according to an embodiment of the present invention;
Figure 2 illustrates a perspective view of the diamond based electrochemical band sensor shown in Figure 1;
Figure 3 illustrates a bottom view of the diamond based electrochemical band sensor shown in Figures 1 and 2;
Figure 4 illustrates a side cross-sectional view of the diamond based electrochemical band sensor shown in Figures 1 to 3;
Figure 5 illustrates a top view of a more complicated multi-functional diamond based electrochemical band sensor according to an embodiment of the present invention;
Figure 6 illustrates a method of manufacturing a diamond based electrochemical band sensor according to an embodiment of the present invention;
Figure 7 illustrates an electrochemical diamond probe according to an embodiment of the present invention;
Figure 8 illustrates a side cross-sectional view of the electrochemical diamond probe according to an embodiment of the present invention;
Figure 9 illustrates a method of mounting a diamond based electrochemical band sensor according to an embodiment of the present invention; and
Figure 10 illustrates an electrochemical cell comprising the electrochemical diamond probe according to an embodiment of the present invention.

### Detailed description of Certain Embodiments

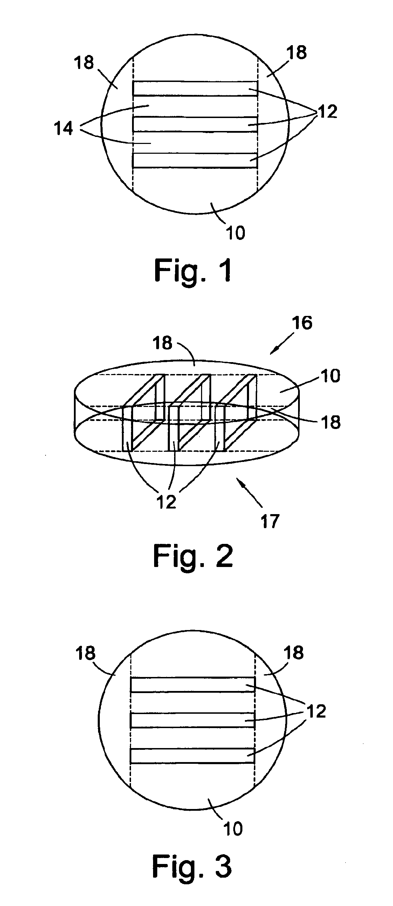

Certain embodiments of the present invention provide a diamond based band electrode as a sensor for electrochemical applications. An example of such a diamond based band electrode is illustrated in Figures 1 to 4.

The electrode comprises a diamond body 10 and a plurality of a plurality of boron doped diamond band electrodes 12 disposed within the diamond body. In the illustrated embodiment three electrodes are shown for simplicity but in practice more electrodes can be provided for multi-functional applications as will be discussed later. Furthermore, it is also envisaged that in more simple applications only one or two band electrodes may be provided.

At least a portion of each of the boron doped diamond band electrodes 12 is doped with boron to a level suitable to achieve metallic conduction, the boron doped diamond electrodes being spaced apart by non-conductive intrinsic layers of diamond 14.

The diamond body 10 comprises a front "sensing" surface 16 with the plurality of boron doped diamond band electrodes 12 being exposed at the sensing surface. The electrodes extend in an elongate manner across the sensing surface to form a plurality of bands. The boron doped diamond electrodes 12 also extend back through the diamond body 10 to a rear "electrical connection" surface 17 of the diamond body opposed to the front sensing surface for providing individual electrical connections to each of the plurality of boron doped diamond band electrodes. Rear electrical connections can be advantageous to avoid contamination or damage of the electrical connection if provided at the front sensing surface. However, for some applications electrical connections at the side surfaces or at the front surface of the sensor may be used.

The boron doped diamond band electrodes 12 thus form a substantially parallel array of boron doped diamond plates extending through the diamond body 10 from the front sensing surface to the rear electrical connection surface. In order to ensure that short circuits between the boron doped diamond band electrodes 12 do not occur along side edges thereof, the side edges should be insulated from one another. In the illustrated embodiment, this is achieved by providing end caps 18 of diamond material adhered to the diamond body 10 adjacent opposing sides of the plurality of boron doped diamond band electrodes (left and right sides of the diamond body as shown in the view illustrated in Figure 1) for insulating side edges of the plurality of boron doped diamond band electrodes from each other. It is possible that these end caps 18 could be made of a material other than diamond or the edges of the band electrodes otherwise insulated from each other by using an insulating material for a holder in which the diamond body is fixed. However, diamond material is preferred for the end caps 18 as this will ensure that the entire sensing surface is made of diamond and is therefore robust to harsh chemical, physical, and thermal environments in which the sensor may be placed. In this arrangement, the boron doped diamond band electrodes are wholly encased in diamond material except at the front sensing surface and the rear surface for forming electrical connections.

Advantageously, the front sensing surface is substantially planar. It has been found that sensing precision is increased by providing a planar sensing surface so as to avoid variations in flux of species at the sensing surface or variations in the electrical response of the sensor during operation. It has also been found that a planar polished surface can provide a good surface for forming metal nanoparticles which adhere well to the surface. By substantially planar it is meant that there are no large steps in height between the adjacent layers of boron-doped and intrinsic diamond on the front sensor surface. That is, such steps are equal to or less than 1 µm, 300 nm, 100 nm, 30 nm, 10 nm, 3 nm, 1 nm, 0.3 nm, or 0.1 nm. In particular, the size of any such step is a factor of 3, 10, 30, 100, 300, or 1000 smaller than the width of the electrode. Preferably the final front sensor surface is polished, to provide an Ra within the same layer exposed on the front sensor surface which is equal to or less than 1.0 µm, 300 nm, 100 nm, 30 nm, 10 nm, 3 nm, or 1 nm. For practical reasons, the Ra is generally equal to or greater than 0.03 nm, 0.1 nm, 0.3 nm, 1.0 nm, 3.0 nm, 10.0 nm, or 30.0 nm. Ra measurements may be made over a line length of at least 10 µm, 20 µm, 50 µm, 100 µm, 500 µm, or 1000 µm. The front sensor surface may however show macroscopic curvature, for example in the form of a segment of a sphere or more preferably a segment of a cylinder, for example to more usefully conform to the wall of a flow tube or flow within the fluid being measured. The radius of curvature of such a cylindrical segment may lie in the range 1 - 100 mm, 2 - 50 mm, 5 - 20 mm, and such macroscopic curvature allowed for in any measurement of the surface Ra, for example by making the measurement parallel to the axis of the cylinder.

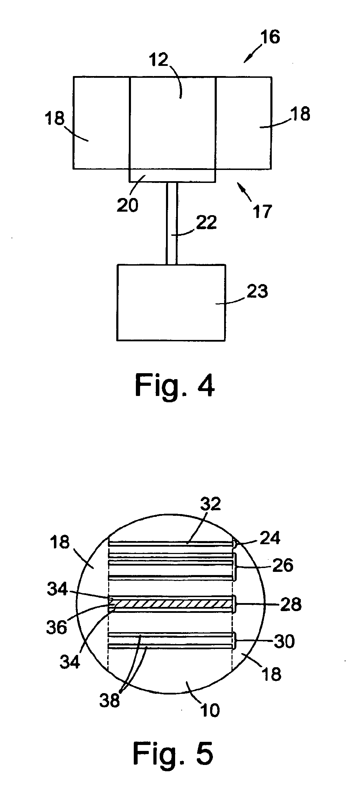

The diamond body and the plurality of boron doped diamond band electrodes may be formed by a single crystal of diamond material or a polycrystalline diamond material. Furthermore, the end caps may be formed by a single crystal diamond material or polycrystalline diamond material. Advantageously, single crystal diamond material is utilized as it considered that this will perform better in many sensing applications. The diamond material is preferably CVD (Chemical Vapour Deposited) diamond material. All, or substantially all, of the front sensing surface may be formed of diamond material so that all, or substantial all, of the sensing surface is mechanically robust and chemically inert. Alternatively, at least 50%, 60%, 70%, 80%, 90%, 95%, or 98% of the sensing surface may be formed of diamond material. The remainder of the sensing surface may be formed of functional material covering one or more of the boron doped diamond electrodes to functionalize the one or more electrodes for a particular application, e.g. IrOₓ for sensing pH as discussed later. If such a functional coating layer is provided on one or more boron doped diamond electrodes at the sensing surface a recess may be provided in which the functional material resides such that the sensing surface remains planar. Alternatively, if the functional coating layer is thin, such a recess may not be required. One or more boron dopes diamond electrodes may also be functionalized with metal nanoparticles, e.g. for sensing H₂S. In some respects, the rear surface of the diamond body as illustrated in Figure 3 is similar to the front sensing surface as illustrated in Figure 1. However, while the sensing surfaces may be treated so as to be planar with a highly polished finish, the rear connection surface of the diamond body need not be of such high quality. The rear surface is required for electrically contacting the individual boron doped diamond band electrodes. To this end, the exposed surfaces of the boron doped diamond band electrodes at the rear surface may be metallized as illustrated in the cross-sectional view shown in Figure 4. A metallization layer 20 is configured to provide individual electrical connections to each boron doped diamond band electrode 12. Wiring 22 may be provided for individually addressing each boron doped diamond electrode 12 and a controller 24 provided in electrical connection with the plurality of boron doped diamond electrodes via the wiring, the controller being configured to individually vary an electrical potential or controlled current applied to each of the plurality of boron doped diamond band electrodes 12 and/or sense changes in potential due to electrochemical reactions.

The embodiment described above and illustrated in Figures 1 to 4 comprises three boron doped diamond band electrodes. However, it is contemplated that a larger number of electrodes are provided within the diamond body to support a range of sensing capabilities. For example, a plurality of boron doped diamond band electrodes may be configured to sense one or more of the following properties of a solution adjacent the sensing surface: pH; conductivity; temperature; individual or total heavy metal concentration; and H₂S. In this regard, the plurality of boron doped diamond band electrodes may be grouped into sets, each set comprising one or more boron doped diamond band electrodes, wherein each set is configured to sense a different parameter.

Such an arrangement is illustrated in the embodiment shown in Figure 5. The illustrated sensor is similar in structure to that shown in Figures 1 to 4 having a diamond body in which a plurality of boron doped diamond electrodes are disposed and end caps at either side of the band electrodes for insulating the side edges of the electrodes from each other. However, this multi-functional embodiment includes fours sets of electrodes 24, 26, 28, and 30 for sensing different parameters. Each set of electrodes is discussed in more detail below.

A first electrode set 24 consists of a single boron doped diamond electrode which has been functionalized with a coating 32 on the sensing surface. Such a functionalized electrode may be utilized for sensing pH of a solution adjacent the sensing surface. An example of a suitable coating material is a noble metal oxide such as an iridium or rhodium oxide. Other alternatives may include an oxide ofPt, Pd, Os, Co, or Ru.

A second electrode set 26 comprises at least two spaced apart boron doped diamond electrodes configured to sense electrical conductivity of a solution disposed adjacent the sensing surface between the electrodes. More preferably, this set may comprise at least three spaced apart boron doped diamond electrodes as illustrated in Figure 5 wherein a spacing between the at least three spaced apart electrodes is different. Providing multiple electrodes with different spacing can aid in providing a more accurate reading for solution electrical conductivity. The specific spacing required can be selected and optimized according to an approximate magnitude of electrical conductivity for solutions to be analysed and/or an address voltage to be applied to the electrodes. By providing multiple electrodes with different spacing it is possible for one sensor to be used in a range of conductive environments. A small spacing might be sufficient for one conductive range whilst a larger spacing would be more sensitive for a different conductive range.

A third electrode set 28 comprises at least two spaced apart boron doped diamond electrodes 34 with a layer of semi-conductive boron doped diamond 36 disposed therebetween to form a sandwich type structure configured to sense at least one of temperature and pressure by sensing the variation in conductivity of the semi-conductive boron doped diamond layer 36 with variations in temperature and/or pressure.

The electrode structure of the third electrode set 28 may also be used to generate heat and thus raise the temperature of the sensing surface. As such, this structure can be used as a sensing element and as an integrated heating element within the diamond body. Alternatively, a separate electrode structure which is the same or similar to electrode set 28 may be provided for use as a heating element. One or more such heating elements may be provided within the diamond body and configured to raise the temperature of the sensing surface sufficiently to clean the sensing surface by increasing the rate of their chemical degradation and removal ("burning off") contaminants adhered to the sensing surface. Alternatively, or additionally, the one or more heating elements may be configured to vary the temperature of the sensing surface to a target value for varying an electrochemical temperature of operation, optionally controlled in a feedback loop from the same or another similar structure measuring the temperature. As such, the diamond based electrochemical band sensor may further comprising a feedback control system to maintain a specific temperature for a predetermined period of time. The high thermal conductivity of diamond permits that the same structure can be used to both generate and measure temperature, these actions occurring either simultaneously or temporally separated in a repeating sequence.

A fourth electrode set 30 comprises at least two spaced apart boron doped diamond electrodes 38 with an intrinsic layer of electrically insulating diamond therebetween. The electrodes 38 are configured to electrochemically deposit and strip heavy metals to determine the heavy metal content in a solution adjacent the sensing surface using stripping voltammetry. These electrodes may be configured to sense one or more heavy metals including, but not limited to, As, Cu, Hg, Cd, and Pb in a solution adjacent the sensing surface. For stripping voltammetry the electrodes may be provided as bare boron doped diamond electrodes. More than two spaced apart and individually addressable electrodes may be provided to sense different heavy metals.

Other electrodes having additional functionality may also be envisaged and may be added to a band electrode array. For example, one of the bands may be configured to generate a proton gradient which flows over one or more of the other bands. This may be used to promote reactions which require a certain pH. In this regard, a diamond electrode material is advantageous in that a very high electrode potential can be applied to alter pH via proton or hydroxide generation. For metal ions which are complexed in solution, digests are normally performed to free them so they are available for subsequent reduction. One way to do this is to generate very strong acid (or base) conditions electrochemically. Furthermore, generating very strong acid (or base) conditions can also be useful for cleaning the electrode.

The previously described electrochemical techniques can also be combined with optical techniques such as spectroscopic measurements by using the non-conductive intrinsic diamond layers as an optical window as described in WO2007/107844.

Figures 1 to 5 are in the form of a circular disk or cylindrical shaped electrochemical sensor. Circular disk shaped sensors have been found to be advantageous as they can be readily mounted in a holder while minimizing stress points in the diamond material to avoid fracture. In this regard, it should be noted that while diamond material is very hard its toughness is not so high and as such it can be susceptible to chipping and/or fracture. The use of a circular disk shaped diamond sensor element can aid in alleviating the problem of chipping and fracture when mounting and using the sensor, and also improve the quality of the seal between it and the mount.

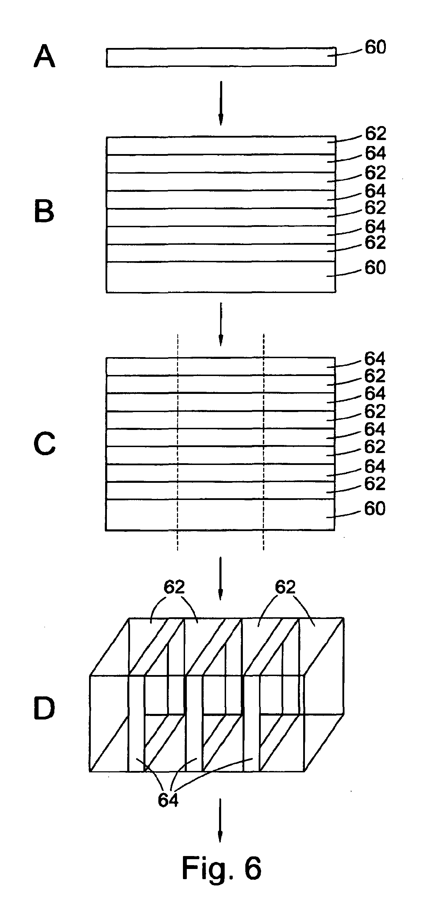

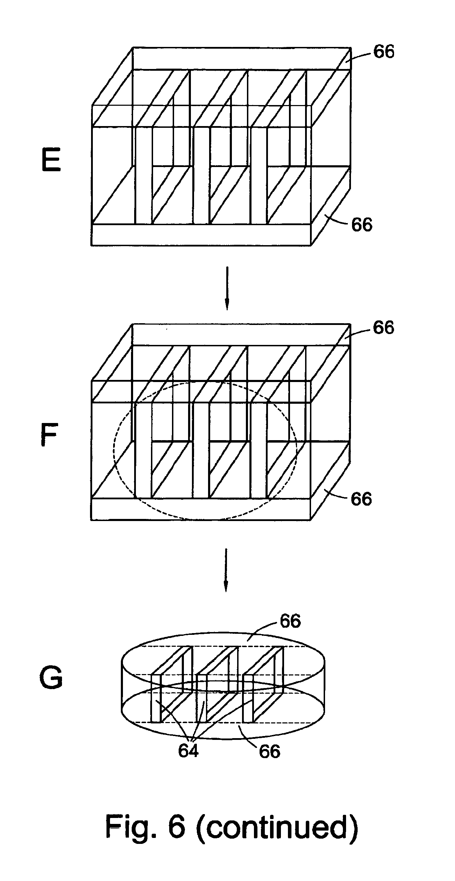

Figure 6 illustrates an example of a method for forming a diamond based electrochemical sensor such as that shown in Figures 1 to 4.

In Step A, a substrate 60 is provided for CVD diamond growth thereon. This substrate 60 may be formed of a single crystal diamond material, particularly if the sensor is to be formed of a single crystal diamond material. Alternatively, if the sensor is to be formed of a polycrystalline diamond material then the substrate 60 may be formed of a metallic material such as tungsten.

In Step B, diamond material is deposited on the substrate 60 using a chemical vapour deposition technique. The diamond material is deposited in a layered structure alternating between layers of intrinsic electrically insulating diamond material 62 and boron doped diamond material 64 which is electrically conductive and which will ultimately form the band electrodes of the electrochemical sensor. CVD diamond growth is optionally performed on a {100}, {110}, or {111} diamond substrate.

In Step C, the layered diamond structure is vertically sliced as indicated by the broken lines and may be rotated by 90° to yield the layered structure shown in Step D. The vertical cutting step may comprise multiple vertical cuts to form a plurality of diamond bodies.

In Step E, end caps 66 are formed on opposing ends of the layered diamond structure to insulate the boron doped diamond layers from each other across two opposing sides of the diamond body such that the boron doped diamond layers are only exposed at two other opposing sides of the diamond body. The end caps may be formed by depositing a layer of diamond over one side, rotating the diamond body 180°, and then depositing a layer of diamond over the opposing side to yield the structure shown in Step E. It should be noted that the end caps may be grown before or after the vertical cutting step to yield a structure such as that illustrated in Step E

The structure shown in Step E may be used as an electrochemical sensor by metallizing one surface at which the boron doped layers are exposed to form electrical connections which may be isolated for each of the boron doped layers and using the opposing surface as a sensing surface for performing electrochemistry. Alternatively, the structure may be cut as illustrated by the broken lines in Step F to form a disk shaped diamond sensor as illustrated in Step G. The structure illustrated in Step G corresponds to that illustrated in Figures 1 to 3. One surface at which the boron doped diamond layers are exposed can be metallized as shown in Figure 4 to form electrical connections to each of the boron doped diamond layers with the opposing surface used as a sensing surface for performing electrochemistry.

The method as described above may be used to form a single crystal or polycrystalline synthetic diamond sensor. Polycrystalline CVD diamond sensors and single crystal diamond sensors are described in more detail below.

The advantage of fabricating sensors in polycrystalline diamond is that the layered structures can be fabricated over large areas, enabling a large number of devices to be part fabricated as one wafer, providing economies of scale

Adjacent layers of boron-doped and intrinsic diamond may be fabricated in a continuous process or in separate growth processes; in either case the grain structure between layers is generally largely continuous, unless deliberate renucleation is initiated for example by use of periods of high methane and/or high Ar in the process, as is known in the art. The grain structure within a thin band exposed at the edge of a layered structure is thus typically one where across the thin direction of the layer (substantially in the direction of growth) the grains are continuous, and along the long dimension (lying in the plane of the original growth surface) grain boundaries are evident giving a characteristic grain size which will be referred to as the 'lateral grain size'. Consequently, using the previously described method of fabrication, in a series of electrode bands the lateral grain size may vary, reflecting the general increase in grain size with growth thickness seen in polycrystalline diamond growth. Generally, this is not detrimental to the performance of the device, provided that the length (lateral extent) of the electrode is significantly larger than the lateral grain size by a factor of at least 10, 20, 30, 50, 100, or 300. This problem is inherently less of an issue than it would be if one used the final growth surface as the electrode surface as this would have the largest grain size.

In other words, using the previously described method the final sensing surface is provided by a cross-section of the as-grown diamond layers. Because the grain size tends to increase during CVD growth of polycrystalline diamond material, variations in grain size will be evident across the sensing surface because the sensing surface is formed by vertically slicing to form a cross-section of the as-grown diamond layers (as illustrated in Figure 6C). As such, each boron doped diamond electrode may have an average lateral grain size, wherein the average lateral grain size increases in a stepwise manner across a series of the boron doped diamond band electrodes. Small grain size in polycrystalline CVD diamond material can be desirable for sensing applications. As such, the fact that all of the sensing surface, or at least the majority of the sensing surface with the exception of one extreme side corresponding to the top surface as-grown (the top layer 64 in Figure 6C which would correspond to a side layer after cutting and rotation by 90° as shown in Figure 6D), is formed of grains which are smaller than the as-grown top surface of the diamond material is advantageous. This contrasts with other methods in which the top-surface of the as-grown CVD diamond material is used as a sensing surface in which case the grains in the sensing surface will be the largest grains in the diamond material. This clearly isn't desirable for certain applications given that sensing functionality can be improved by providing smaller grains.

Typically the lateral grain size may be equal to or less than 300 µm, 100 µm, 50 µm, 30 µm, or 10 µm, and/or equal to or greater than 0.1 µm, 0.2 µm, 0.5 µm, 1 µm, 2 µm, or 5 µm. It should be noted that the lateral grain size can be calculated as an average grain size along the length of an electrode. This can simply be measured by taking an image of an electrode, drawing a line of specified length, counting the number of grain boundaries along the line, and then dividing the length of the line by the number of grain boundaries to yield an average grain size. Alternatively the line selected could be that defined by the boundary between the boron doped layer and another layer of the structure.

It may be preferable to process the diamond between forming successive layers to generate good flat edge definition between layers. Such processing may include techniques such as lapping and polishing which are known in the art. This can be characterised by the Ra measured along a line, either measured on the exposed surface of the diamond before a next layer is added, or by characterising the cross-sectional line of this interface exposed by the forming the front sensing surface through the layers to provide the final device structure. Preferably the interface between the intrinsic and boron doped layers shows an Ra which is equal to or less than 10.0 µm, 5.0 µm, 2.0 µm, 1.0 µm, 0.5 µm, 0.2 µm, 0.1 µm, 0.05 µm, or 0.02 µm. For practical reasons, the Ra is generally equal to or greater than 0.2 nm, 0.5 nm, 1.0 nm, 2.0 nm, 5.0 nm, or 10.0 nm. Ra measurements may be made over a line length of at least 10 µm, 20 µm, 50 µm, 100 µm, 500 µm, or 1000 µm. Since the overgrowth of a polycrystalline layer which has been surface processed to flat generally re-establishes the granular surface morphology of an unprocessed layer, it is generally better to process the diamond between each layer, although some applications may not require this.

Boron uptake, and thus electrical conductivity in the diamond material, is a function of the growth sector, with the highest boron uptake generally in the {111} growth sector followed by the {110} growth sector and then the {100} growth sector. In order to form highly conductive diamond with a high boron concentration, it can be advantageous to select growth conditions to enhance the formation of {111} growth facets during the doped diamond growth. Note that this is not necessarily the same as establishing a <111> wire texture in the polycrystalline layer.

The dislocation structure in individual polycrystalline diamond grains is complex. None the less, the overall direction of dislocations in polycrystalline growth is in the direction of growth. A further advantage of forming the final electrode surface by severing across a boron doped diamond layer is that it reduces the number of dislocations which break the sensor surface, which can improve the electrochemical performance of the electrode. The growth direction in a polycrystalline layer in the sensor structure can be determined from the grain morphology and the orientation of interfaces between layers. Preferably the angle between the growth direction and the normal to the plane of the final sensor surface is greater than 45°, 60°, 70°, 75°, 80°, or 85°.

In contrast to the polycrystalline CVD diamond sensors discussed above, the advantage of using single crystal diamond is that there are no grain boundaries, that the material forming the final sensor surface can have a much lower density of extended defects, that the crystallographic direction of the growth direction (and thus the growth sector of the material) can be well defined, that the crystallographic orientation of the final sensor surface can be well defined, and that the boron concentration uniformity can be achieved on a much smaller scale, enabling smaller electrodes. These factors combined may enable a higher performance electrode to be fabricated for particular applications.

Adjacent layers of boron and intrinsic diamond may be fabricated in a continuous process or in separate growth processes. It may be preferable to process the single crystal diamond between layers to generate good flat edge definition between layers. Such processing may include techniques such as lapping and polishing which are known in the art. This can be characterised by the Ra measured along a line, either measured on the exposed surface of the diamond before a next layer is added, or by characterising the cross-sectional line of this interface exposed by the forming the surface through the layers which provides the final device structure. Preferably the interface between the intrinsic and boron doped layers shows an Ra which is equal to or less than 1.0 µm, 0.5 µm, 0.2 µm, 0.1 µm, 0.05 µm, or 0.02 µm. For practical reasons, the Ra is generally equal to or greater than 0.1 nm, 0.2 nm, 0.5 nm, 1.0 nm, 2.0 nm, 5.0 nm, or 10.0 nm. Ra measurements may be taken over a line length of at least 10 µm, 20 µm, 50 µm, 100 µm, 500 µm, or 1000 µm. Alternatively, the single crystal growth process can be selected to minimise the development of surface roughness during growth, to enable the deposition of a series of two or more layers without intervening processing steps. To assist in this it is beneficial to control the offset angle between the major face defining the intended growth sector and the actual orientation of the growth surface, for example as measured at the interface between a boron doped layer and an intrinsic layer, in a range 0.1° to 5°, 0.1° to 3°, 0.1° to 2°, 0.1° to 1°, or 0.5° to 1°, where the major face is one of {100}, {110}, {111}.

Boron uptake, and thus electrical conductivity in the diamond material, is a function of the growth sector, with the highest boron uptake generally in the {111} growth sector followed by the {110} growth sector and then the {100} growth sector. In order to form highly conductive diamond with a high boron concentration, it can be advantageous to select growth conditions to enhance the formation of {111} growth facets during the doped diamond growth. It is also important to minimise the formation of secondary growth facets (e.g. associated with pits) as these will locally modify the boron uptake. The device may preferentially be formed from material which is predominantly (equal to or greater than 50%, 60%, 70%, 80%, 90%, or 95% by volume) one of the following growth sectors: {111}, {110}, or {100}, where this requires the growth surface to have been within 10°, 5°, 3°, 2°, or 1° of the relevant orientation. Furthermore, the front sensing surface may lie within 10°, 5°, 3°, 2°, or 1° of a {111}, {110}, or {100} crystallographic plane. There is an advantage in using a {111} growth sector because of its very high boron uptake. However, it can also be advantageous to use material formed predominantly from a {110} growth sector as this enhances boron uptake compared to {100}, has lower uptake of other defects compared to {111}, and enables the formation of sensor surfaces whose normal is at 90° to the growth direction from any of the major planes {100}, {110}, or {111}. It has also been found that for certain so called "inner-sphere" redox reactions a front sensing surface having a {100} orientation is preferred. The dominant oxygen functional groups on a {100} diamond face are expected to be C=O or C-O-O, whereas for the {110} and {111} faces, C-OH groups are likely to dominate. Cyclic voltammograms obtained for different crystallographic orientations imply that upon anodic polarisation, C=O groups are generated at a {100} diamond face, facilitating electron transfer. However, these groups are not created to such an extent at the {110}, {111} or polycrystalline surfaces, leaving a low density of active sites. This would suggest that by use of single crystal diamond, a high number of specific active site could be created in order to facilitate electron transfer to certain redox species by using {100} oriented single crystal material.

The dislocation direction in single crystal diamond generally follows the growth direction. A further advantage of forming the final electrode surface by severing across a boron doped diamond layer is that it reduces the number of dislocations which break the sensor surface, which can improve the electrochemical performance of the electrode. The growth direction in a single crystal diamond layer can be determined from the dislocation structure and the orientation of interfaces between layers. Preferably the angle between the growth direction and the normal to the plane of the final sensor surface is equal to or greater than 45°, 60°, 70°, 75°, 80°, or 85°.

Alternative methods of manufacture are also possible. For example, a hybrid single crystal - polycrystalline diamond sensor may be formed by, for example, providing a plurality of boron doped single crystal diamond electrodes in an array, growing non-conductive diamond material over and between the array of boron doped single crystal diamond electrodes, and then processing back the non-conductive diamond material to expose the plurality of boron doped single crystal diamond electrodes thus forming a front sensing surface with single crystal boron doped diamond electrodes embedded in a non-conducting diamond matrix. In this method, polycrystalline diamond material may grow between the boron doped diamond electrodes with either single crystalline or polycrystalline diamond material being formed over the boron doped diamond electrodes. The nature of the material deposited over the boron doped diamond electrodes is not as important as this material can be processed back to reveal the boron doped diamond electrodes. The composite structure may then be removed from an underlying carrier substrate to expose the plurality of boron doped single crystal diamond electrodes at a rear electrical connection surface and yield a free-standing sensor as previously described.

In an alternative to the aforementioned method, the growth surface may be used as the electrical connection surface and the rear surface may be used as the sensing surface.

Another possible method of manufacture may involve etching a surface electrode structure onto a boron doped diamond body, growing non-conductive material over the surface electrode structure, and then processing back either the boron doped diamond or the non-conductive material to expose the surface electrode structure of the boron doped diamond.

It will be noted that the methods as described above can lead to a sensor structure in which the boron doped diamond band electrodes may extend all the way through the diamond body from the front sensing surface to the rear electrical connection surface. Alternatively, the methods as described above can be used to manufacture a sensor in which the boron doped diamond band electrodes extend only part-way through the diamond body from the front sensing surface to the rear electrical connection surface. In that case, openings may be formed in the rear electrical connection surface extending from the rear electrical connection surface to the boron doped diamond electrodes within the diamond body for providing individual electrical connections to each of the plurality of boron doped diamond electrodes.

It will also be noted that while the embodiments described above may consist of only linear boron doped diamond electrodes extending over the sensing surface, it is also possible to provide additional boron doped diamond electrodes which are non-linear. For example, the sensor may comprise a parallel array of boron doped diamond band electrodes in a central region of the sensing surface and curved electrodes around a periphery of the sensing surface.

Thus far, the basic structure of a diamond based electrochemical band sensor and suitable methods for manufacturing the same have been described. While the specific dimensions of such a diamond based electrochemical band sensor may be selected according to a desired end use, it has been found that certain design rules may be advantageously followed in order to achieve better functional performance. In particular, it has been found that it is highly advantageous to provide band electrodes which have a high aspect ratio at the sensing surface such that a length of a band electrode across the sensing surface is very much larger than a width of the band electrode. As such, according to certain preferred arrangements it is desirable that each boron doped diamond electrode has a length / width ratio of at least 10, 20, 30, 40, 50, 100, 500, 1000, 2000, 5000, or 8000 at the sensing surface.

From a functional point of view the length/width ratio may be as large as possible. However, practical considerations in manufacturing the electrodes may limit the size of the length / width ratio of the electrodes. Accordingly, in practice the length / width ratio may optionally be equal to or less than 15000, 10000, 8000, 5000, 2000, or 1000 depending on the manufacturing technique which is used. For example, the length / width ratio may lie in a range 10 to 15000, 20 to 10000, 30 to 5000, or 50 to 1000.

For certain applications each boron doped diamond electrode may have a width of at least 0.1 µm, 0.5 µm, 1 µm, 2 µm, 5 µm, 10 µm, or 15 µm. The width of each electrode may be equal to or less than 100 µm, 80 µm, 60 µm, 40 µm, 20 µm, 10 µm, 3 µm, or 1 µm. For example, the width may lie in a range 0.1 to 100 µm, 1 to 80 µm, 5 to 60 µm, 10 to 40 µm, or 15 to 30 µm.

For certain applications each boron doped diamond electrode may have a length of at least 100 µm, 200 µm, 400 µm, 600 µm, 800 µm, or 1000 µm. The length of each electrode may be equal to or less than 10000 µm, 8000 µm, 6000 µm, 4000 µm, or 2000 µm. For example, the length may lie in a range 100 to 10000 µm, 200 to 8000 µm, 400 to 6000 µm, 600 to 4000 µm, 800 to 2000 µm, or 1000 to 2000 µm.

For certain applications each electrode may have a surface area at the sensing surface of at least 0.0001 mm², 0.001 mm², 0.005 mm², 0.010 mm², 0.015 mm², or 0.020 mm². The surface area of each electrode may be equal to or less than 1.000 mm², 0.500 mm², 0.100 mm², 0.050 mm², or 0.030 mm². For example, the surface are may lie in a range 0.0001 mm² to 1.000 mm², 0.001 mm² to 0.500 mm², 0.005 mm² to 0.100 mm², 0.010 mm² to 0.050 mm², 0.015 mm² to 0.030 mm².

The spacing between the boron doped diamond electrodes is optionally larger than the width of each boron doped diamond electrode. For example, a ratio of electrode spacing / electrode width may be at least 1, 1.5, 2, 4, 6, 8, 10, 50, 100, 500, or 1000. The ratio of electrode spacing / electrode width may be equal to or less than 10000, 5000, 1000, 500, 100, 50, 40, 30, 20 or 15. For example the ratio of electrode spacing / electrode width may lie in a range 1.5 to 10000, 4 to 5000, 10 to 1000, 40 to 500, or 50 to 100.

In relation to the dimensions recited above, it should be noted that different boron doped diamond electrodes within a single sensor may have different dimensions. For some applications this may be important. For example, in generation and collection applications it may be desirable to generate species from a thin electrode and collect species at a thicker electrode.

In relation to the dimensions recited above, it may also be noted that increasing electrode length and decreasing electrode spacing can reduce capacitance. Reduced capacitance allows improved electrochemical sensitivity and can be useful in enabling lower solution conductivities to be measured. As such, a band electrode structure can be advantageous in this regard. Furthermore, a high ac operating frequency can also be used to reduce capacitive effects, e.g. an operating frequency of 5 kHz, 10 kHz or more may be desirable. Detrimental capacitive effects can thus be reduced to increase sensitivity by: (i) use of high ac frequency; and (ii) selected band electrode geometry.

The boron doped diamond electrodes may extend through the diamond body from a front sensing surface to a rear electrical connection surface of the sensor. A side surface of the sensor extends from the front sensing surface to the rear electrical connection surface around the boron doped diamond electrodes. The boron doped diamond electrodes may be sufficiently spaced apart from the side surface of the sensor so as to prevent short circuits via the side surface. As such, the boron doped diamond electrodes form an array which extends through a central region of the sensor with a peripheral region of non-conductive material. A minimum distance between the boron doped diamond electrode array and the side surface of the sensor may be selected to avoid short circuits. This minimum distance will depend on the operating voltage to be applied to the boron doped diamond electrodes but will typically be at least 10 µm, 20 µm, 40 µm, 60 µm, 80 µm, or 100 µm. The minimum distance may be larger still although this will require an increase in the overall size of the sensor relative to the size of the boron doped diamond electrodes such that only a relatively small portion of the sensing surface is active. Accordingly, for efficiency of manufacture and use of material the aforementioned minimum distance may be selected so as to be not unduly large. For example, the minimum distance may be equal to or less than 5 mm, 3 mm, 2 mm, 1 mm, 0.5 mm, or 0.2 mm.

For best functional performance, the boron doped diamond electrodes should have a precise, uniform, and reproducible geometry. Using the method of manufacture as illustrated in Figure 6, the length of the electrodes can be precisely defined by vertically slicing the as grown diamond material comprising the layers of boron doped diamond which are to form the electrodes in the sensor (see, for example, Figure 6C). The side surface may be further processed if required to provide a precisely defined electrode length. However, the width of the boron doped diamond electrodes will be determined by the growth conditions which must be carefully controlled to form boron doped diamond layers have a desired and uniform thickness which corresponds to the width of the electrodes in the final sensor. Preferably, the growth conditions are controlled so each boron doped diamond electrode has a width which varies by no more than ±20 µm, ±15 µm, ±10 µm, ±5 µm, ±2.5 µm, ±1 µm, ±0.1 µm, ±0.02 µm, ±0.005 nm, ±0.001 µm of a mean width along the length of the electrode. In this regard, it should be noted that it is difficult to form very well defined boron doped layers in a single CVD growth run. For example, it is difficult to sharply cut off the boron dopant being taken up by the synthetic CVD diamond material during a single growth run. One approach to solve this problem is to grow a boron doped layer in one growth run and then transfer the material into another reactor to grow undoped material thereon. This allows the width of the boron doped layers and thus the width of the resultant electrodes in the final sensor to be more precisely defined. Alternatively, it has been found that high velocity process gas flow directed towards the growth surface can be used to more quickly ramp up and ramp down boron uptake in a single growth run to form boron doped diamond layers having a well defined and uniform thickness. Optionally, a multi-nozzle gas inlet may also be utilized to achieve high velocity, uniform process gas flow towards the growth surface of the CVD diamond material during synthesis.

Using one of the aforementioned methods is it possible to form a layered CVD diamond structure in which the boron dopant can vary by at least a factor of 3, 10, 30, 100, 300, 1000, 30000, or 100000 over a distance of no more than 10 µm, 3 µm, 1 µm, 0.3 µm, 0.1 µm, 0.03 µm, 0.01 µm, 0.003 µm, or 0.001 µm. Embodiments thus allow the concentration of boron to be changed quickly through a thickness of as grown CVD diamond material to form precisely defined boron doped diamond electrodes. If the layers of boron doped and undoped diamond material are formed in a single growth run it is possible to avoid contamination of interfaces between the layers which may result from transferring the diamond material between different reactors. Accordingly, interfaces between the boron doped diamond band electrodes and non-conductive intrinsic layers of diamond may be substantially free of impurities whereby, in a region either side of an interface extending to 20%, 50%, or 100% of a thickness of an associated boron doped diamond band electrode, an impurity concentration does not exceed 10¹⁴, 3 x 10¹⁴, 10¹⁵, 3 x 10¹⁵, 10¹⁶, 3 x 10¹⁶, or 10¹⁷, and does not vary in concentration by more than a factor of 2, 3, 5, 10, 30, 100, 300, or 1000.

As an alternative or in addition to the above, the sensor may be provided with a controller which is configured to compensate for variations in the width of the boron doped diamond electrodes (and optionally other design parameters which may vary according to manufacturing tolerances). That is, the sensors may be calibrated to account for geometric variations in order to provide a consistent functional performance and thus a more reliable commercial product.

In addition to the above mentioned geometric considerations, the functional performance of the sensor will depend on the concentration and uniformity of boron dopant within the boron doped diamond electrodes. At least a portion of each boron doped diamond electrode may comprise a boron concentration equal to or greater than 1 x 10²⁰ cm⁻³ 2 x 10²⁰ cm⁻³, 4 x 10²⁰ cm⁻³, 5 x 10²⁰ cm⁻³, 7 x 10²⁰ cm⁻³, 1 x 10²¹ cm⁻³, or 2 x 10²¹ cm⁻³. Preferably, the concentration of boron varies by no more than 50%, 30%, 20%, 10%, or 5% of a mean concentration over at least 70% 80%, 90%, or 95% of the area of the boron doped diamond electrode at the sensing surface, at least for single crystal boron doped diamond electrodes. For the semi-conductive boron doped diamond layer 36 illustrated in Figure 5, lower concentrations of boron may be utilized. For example a boron concentration in a range 10¹⁶ to 10¹⁹ or 10¹⁷ to 10¹⁸ may be utilized to form such a semi-conductive layer.

For polycrystalline boron doped diamond electrodes, it has been found that only a portion of the material is required to be doped to a sufficient level to be metallically conductive. As such, for polycrystalline boron doped diamond electrodes only a portion of grains at an exposed working surface of each electrode are required to be metallically conductive having a boron content of at least 1 x 10²⁰ boron atoms cm⁻³. In contrast, for single crystal boron doped diamond electrodes it is advantageous that substantially all the exposed working surface of each electrode is doped to such a level.

The functional performance of the sensor may also be detrimentally affected by the presence of dopants other than boron. Accordingly, the boron doped diamond electrodes may comprise a dopant ratio x / y equal to or greater than 2, 5, 10, 20, 50, 100, 500, or 1000, where x is boron and y is unintentional dopant which may be present as an impurity in the process gas. For example, y may be nitrogen or silicon.

Further still, the functional performance of single crystal diamond sensors can be detrimentally affected by the presence of extended defects such as dislocations. Accordingly, the boron dopant is advantageously introduced into single crystal CVD diamond material while maintaining low levels of extended defects such as dislocations. As such, the boron doped diamond electrodes may comprise a concentration of dislocation features no more than 10⁵, 10⁴, or 10³ cm⁻². The concentration of extended defects can be controlled by careful selection and processing of substrates prior to synthesis thereon, and optionally processing the growth surface to reduce surface roughness between growth of alternating layers.

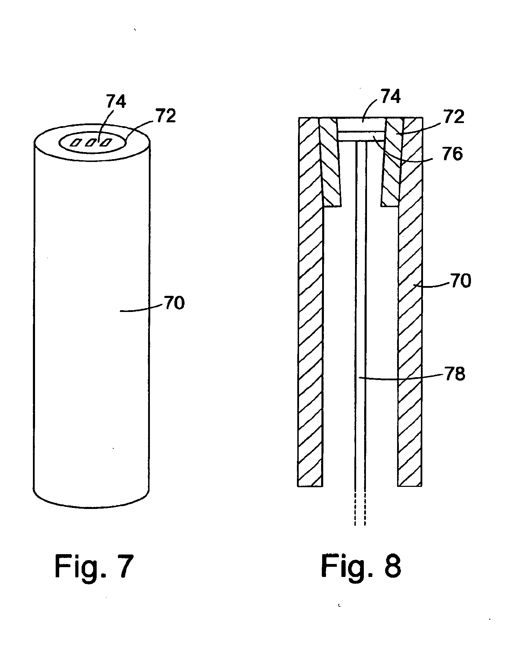

A diamond sensor such as one formed as described previously may be mounted to form an electrochemical probe. An example of such a probe is illustrated in Figures 7 and 8. The electrochemical probe may comprise:
a tubular body 70 defining an internal channel and an opening;
a mounting ring 72 which is mounted within the internal channel and configured to define an aperture aligned with the opening; and
an electrochemical diamond sensor 74 bonded to the mounting ring within the aperture, preferably using a braze join,
wherein the mounting ring 72 comprises a material having a coefficient of linear thermal expansion α of 14 x 10⁻⁶ K⁻¹ or less at 20°C and a thermal conductivity of 60 Wm⁻¹K⁻¹ or more at 20°C, and
wherein optionally the tubular body 70 is made of a chemically inert material.

As shown in the cross-sectional view of Figure 8, metallization 76 may be provided on a rear side of the diamond sensor and wiring 78 connected to the metallization for providing electrical connections to the boron doped diamond electrodes within the diamond sensor.

The present inventors have realized that to provide a chemically inert electrochemical probe with a diamond sensor requires both a chemically inert tubular body and a mounting ring which is made of a material having a coefficient of linear thermal expansion α of 14 x 10⁻⁶ K⁻¹ or less at 20°C and a thermal conductivity of 60 Wm⁻¹K⁻¹ or more at 20°C. The mounting ring is mounted within the chemically inert tubular body and the diamond sensor is bonded to the mounting ring. As such, the chemically inert tubular body protects the mounting ring from the external chemical environment and the mounting ring provides a reliable bonding to the diamond sensor to prevent delamination. If any exposed areas of the mounting ring remain around the diamond window they can be coated with an inert material, which is preferably electrically insulating, to prevent adverse reactions.

The coefficient of linear thermal expansion α is advantageously 12 x 10⁻⁶ K⁻¹ or less, 10 x 10⁻⁶ K⁻¹ or less, 8 x 10⁻⁶ K⁻¹ or less, 6 x 10⁻⁶ K⁻¹ or less, or 4 x 10⁻⁶ K⁻¹ or less.

The thermal conductivity is advantageously 60 Wm⁻¹K⁻¹ or more, 80 Wm⁻¹K⁻¹ or more, 100 Wm⁻¹K⁻¹ or more, 120 Wm⁻¹K⁻¹ or more, or 140 Wm⁻¹K⁻¹ or more.

The mounting ring may be formed of at least 50% of the low thermal expansion coefficient/high thermal conductivity material. More preferably, the mounting ring is formed of at least 70% of said material, at least 80% of said material, at least 90% of said material, or at least 95% of said material. The material may be a metal, an alloy, a ceramic, or a composite material. Examples of such materials include one or more of molybdenum, chromium, tungsten, nickel, rhodium, ruthenium, silicon carbide (SiC), tungsten carbide (WC), aluminium nitride (AlN), molybdenum alloys such as titanium zirconium molybdenium (TZM), and tungsten alloys such as tungsten nickel iron (WNiFe) and tungsten nickel copper (WNiCu). Another possibility is to manufacture the mounting ring from a diamond material such as polycrystalline CVD diamond.

Molybdenum has been found to be particularly useful as it can readily be manufactured into a mounting ring, has a low thermal expansion coefficient of 5 x 10⁻⁶ K⁻¹, and has a relatively high thermal conductivity of 144 Wm⁻¹K⁻¹.

The mounting ring may be a relatively flat ring-shaped member, somewhat like a washer. However, in order to thermally conduct heat away from the diamond sensor it may be advantageous to make the mounting ring more elongate or tubular in shape. The mounting ring may also have a tapered inner surface for receiving electrical wiring.

The mounting ring may be mounted within the chemically inert tubular body in a variety of ways. In one preferred configuration the chemically inert tubular body comprises an internally tapered end portion and the mounting ring is press-fit into the tapered end portion. However, other arrangements are envisaged. For example, the opening of the tubular body may be disposed in an end or in a side wall of the tubular body with the mounting ring suitable mounted within the tubular body so as to define an aperture which is aligned with the opening.

By providing a mounting ring made of a material which is selected to achieve reliable bonding to the diamond sensor, the tubular body can then be selected from a range of chemically inert materials according to the probes intended use. By chemically inert, we mean that the tubular body is more chemically inert than the mounting ring. For example, the tubular body may be made of a material which is less reactive to acidic environments than the mounting ring. Example materials include inert and/or corrosion resistant alloys, ceramics, or composite materials. Nickel alloys have been found to be useful and particularly the commercially available Hastelloy™ superalloys such as Hastelloy C-276, a corrosion resistant Nickel-Molybdenum-Chromium alloy with addition of Tungsten. However, it will be understood that the chemically inert material may be selected according to the end use of the electrochemical probe.

The chemically inert tubular body may be selected to have a variety of possible shapes according to its intended use. For many applications a circular or oval cross-sectional shape will be suitable. The largest external diameter of the tubular member may be 30 mm or less, 20 mm or less, 15 mm or less, or 10 mm or less. The term "largest external diameter" is simply the diameter when the tubular body has a circular cross section. When the tubular member has a non-circular cross-section, such as an oval cross-section, then the largest external diameter refers to the largest distance across the cross-section in a direction perpendicular to a longitudinal axis of the tubular body.

The tubular body may have a wall thickness of 0.5 mm to 4 mm, 0.5 mm to 3.0 mm, 0.5 mm to 2.0 mm, 0.7 mm to 1.2 mm, or 0.8 mm to 1.0 mm. Furthermore, the internal diameter of the tubular member may be 0.2 mm to 20 mm, 0.3 mm to 15 mm, 0.4 mm to 10 mm, or 0.5 mm to 5 mm. Diamond sensor de-bonding may be problematic for such a small chemically inert tubular body unless provided with an internal mounting ring according to the present invention.

The diamond sensor may be provided with a metallization coating on an internal surface in an area around the aperture. Such a coating aids bonding between the diamond sensor and the tubular body. A preferred metallization coating comprises a layer of a carbide forming material such as titanium, an inert barrier layer such as platinum, and a metal layer such as gold for soldering or brazing to the tubular body gold. The titanium provides a good bond with the diamond forming titanium carbide at an interface with the diamond. The gold provides a good bond to the tubular body. The platinum functions as an inert shield between the gold and titanium.

A braze join can be provided between the metallization coating and the mounting ring for bonding the diamond sensor to the tubular body. The braze join may comprise gold. A gold-tantalum braze has been found to be useful as it is chemically inert and non-corrosive. The braze material can extend around the periphery of the diamond sensor to cover any exposed portions of the mounting ring around the diamond sensor and prevent adverse reaction of the mounting ring material with the surrounding chemical environment in use.

The present invention is particularly useful when applied to a small diamond sensor which is more susceptible to heating. Accordingly, the diamond sensor may have a thickness in the range 0.1 mm to 5 mm, 0.1 mm to 2 mm, 0.1 mm to 1 mm, 0.1 mm to 0.5 mm, 0.1 mm to 0.3 mm, 0.1 mm to 0.2 mm, or 0.1 mm to 0.2 mm. Furthermore, the diamond sensor may have a longest dimension in the range 1.0 mm to 10.0 mm, 1.0 mm to 8.0 mm, 1.5 mm to 5.0 mm, or 2.5 mm to 3.5 mm. Further still, the diamond sensor may have a width in the range 1.0 mm to 5.0 mm, 1.0 mm to 3 mm, or 1.5 mm to 2.5 mm. The diamond body of the sensor may have a thickness and a largest lateral dimension whereby a ratio thickness : largest lateral dimension is in a range 1 : 10 to 1.5 : 1; 1 : 5 to 1 : 1; or 1 : 3 to 1 : 2.

One further problem which the present inventors have identified is that the diamond sensor can be damaged if it is knocked by an external member, such as a stirring apparatus within a chemical reactor in which the probe is placed. As such, it has been found to be advantageous to position the diamond window in a recess of the tubular body such that at least a portion of the tubular body extends beyond the diamond sensor to protect it from damage. The diamond sensor may thus be disposed in a recess across the aperture. This arrangement ensures that the diamond window extends across the entire aperture while being wholly located within the recess such that that diamond window is protected.

The aforementioned recess may be formed by a plurality of projections with a plurality of openings disposed therebetween around the diamond sensor. The openings allow fluid to flow more readily into and out of the recess so that fluid adjacent to the diamond window is representative of the composition of a sample which is being analysed. Without such openings, it has been found that fluid can become trapped in the recess such that it doesn't mix and react with other components in the sample resulting in misleading readings.

Alternatively the diamond sensor may be mounted flush or slightly exposed from the mount, to facilitate mechanical cleaning.

The diamond sensor is preferably bonded to the mounting ring around the aperture to form a seal all around the aperture. This will prevent fluid or other debris entering the tubular body and fouling the electrical connections within the probe.

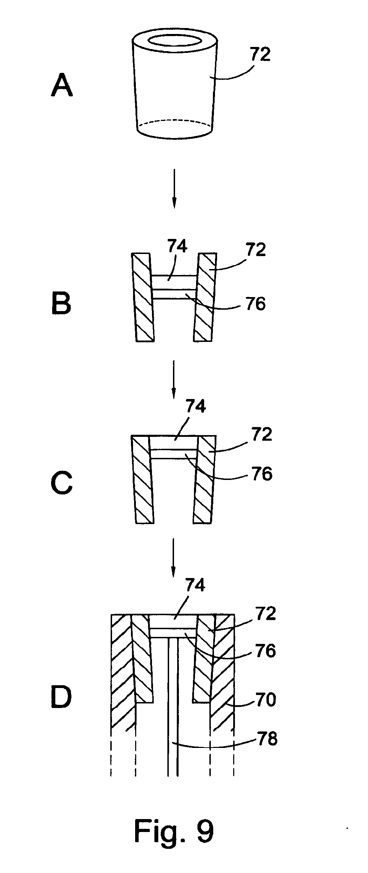

Figure 9 shows a method for mounting a diamond sensor in an electrochemical probe according to an embodiment of the present invention.

In Step A, a mounting ring 72 is provided. In Step B, a diamond sensor 74 is push fit into the mounting ring 72. The diamond sensor 74 may be bonded to the mounting ring 72 using, for example, a braze join. The sensor may be provided with metallization 76 on a rear surface therefore for electrically connecting the boron doped diamond electrodes within the sensor. The metallization can be performed via electroplating using the boron doped diamond band electrodes to achieve self-alignment of the electrical contacts and the boron doped diamond band electrodes.

In Step C, the top portions of the mounting ring are removed to form a surface which is planar with a sensing surface of the diamond sensor. As such, the mounting ring functions as a holder for the diamond body. Alignment of these components is aided by mounting the diamond body in a holder having a bevelled edge and removing portions of the holder extending beyond the sensing surface such that the holder and the diamond sensor form a flush flat surface.

Finally, in Step D, the mounting ring and diamond sensor arrangement can be push-fit into a tubular body (or potentially some other shaped holder) to form an electrochemical probe with wiring 78 connecting to the boron doped diamond electrodes within the sensor.

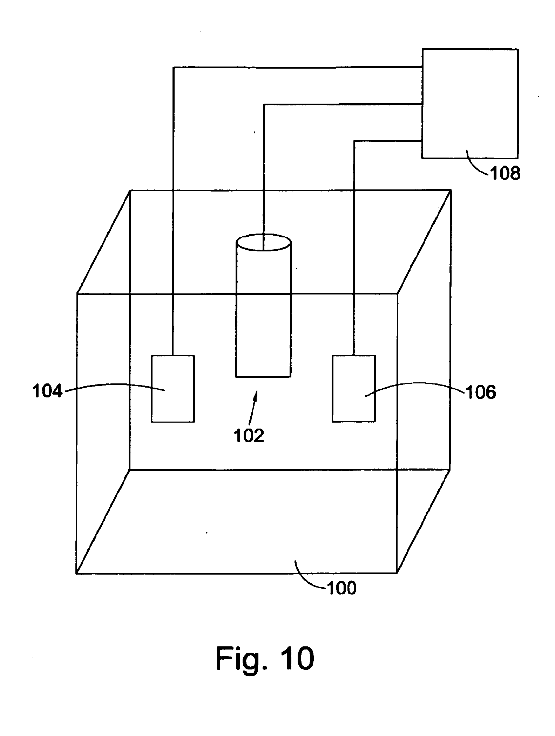

In use, the electrochemical sensor may be introduced into an electrochemical cell as shown in Figure 10. The electrochemical cell comprises a housing 100 in which a solution which is to be analysed is disposed. In the illustrated embodiment, the diamond sensor is disposed in an electrochemical probe 102 as illustrated in Figures 8 and 9. Also provided in the electrochemical cell are a counter electrode 104 and a reference electrode 106. These electrodes are connected to a controller 108 for applying a potential to the electrodes and/or sensing a potential generated at the electrodes by an electrochemical reaction. In one advantageous arrangement the counter electrode and the reference electrode can be made of diamond material and integrated into the diamond sensor.

Furthermore, in use the electrode may be in contact with fluid which is flowing, and which has a distinct flow direction. Generally the front sensor surface of the device will be oriented such that the flow direction lies substantially parallel to the front sensor surface, and if the front sensor surface is curved in the form of a cylinder then the flow direction lies parallel to the cylindrical axis. In some applications the orientation of the long axis of the electrodes may be at an arbitrary angle with respect to the flow direction. In others it may beneficial to orient the long axis of the electrodes substantially perpendicular to the flow direction, i.e. at > 70°, 80°, 85° to the flow direction, and in other applications it may be beneficial to orient the long axis of the electrodes substantially parallel to the flow direction, i.e. < 20°, 10°, 5° to the flow direction.

## Claims

1. A diamond based electrochemical band sensor comprising:
a diamond body (10); and
a plurality of boron doped diamond band electrodes (12) disposed within the diamond body,
wherein at least a portion of each of the plurality of boron doped diamond band electrodes is doped with boron to a level suitable to achieve metallic conduction, the boron doped diamond electrodes being spaced apart by non-conductive intrinsic layers of diamond (14),
wherein the diamond body (10) comprises a front sensing surface (16) with the plurality of boron doped diamond band electrodes (12) being exposed at said sensing surface and extending in an elongate manner across said surface, and
**characterised in that** each boron doped diamond electrode (12) has a length / width ratio of at least 10 at the front sensing surface (16).

2. A diamond based electrochemical band sensor according to claim 1, wherein the diamond body has a thickness and a largest lateral dimension whereby a ratio thickness : largest lateral dimension is in a range 1 : 10 to 1.5 : 1; 1 : 5 to 1 : 1; or 1 : 3 to 1 : 2.

3. A diamond based electrochemical band sensor according to claim 1 or 2, wherein each boron doped diamond electrode has a length / width ratio of at least 20, 30, 40, 50, 100, 500, 1000, 2000, 5000, or 8000 at the front sensing surface.

4. A diamond based electrochemical band sensor according to claim 3, wherein the length / width ratio is no more than 15000, 10000, 8000, 5000, 2000, or 1000.

5. A diamond based electrochemical band sensor according to claim 3 or 4, wherein the length / width ratio is in a range 10 to 15000, 20 to 10000, 30 to 5000, or 50 to 1000.

6. A diamond based electrochemical band sensor according to any preceding claim, wherein each boron doped diamond electrode has a width of at least 0.1 µm, 0.5 µm, 1 µm, 2 µm, 5 µm, 10 µm, or 15 µm.

7. A diamond based electrochemical band sensor according to claim 6, wherein the width of each electrode is equal to or less than 100 µm, 80 µm, 60 µm, 40 µm, 20 µm, 10 µm, 1 µm, or 0.5 µm.

8. A diamond based electrochemical band sensor according to claim 6 or 7, wherein the width of each electrode is in a range 0.1 to 100 µm, 1 to 80 µm, 5 to 60 µm, 10 to 40 µm, or 15 to 30 µm.

9. A diamond based electrochemical band sensor according to any preceding claim, wherein each boron doped diamond electrode has a length of at least 100 µm, 200 µm, 400 µm, 600 µm, 800 µm, or 1000 µm.

10. A diamond based electrochemical band sensor according to claim 9, wherein the length of each electrode is equal to or less than 10000 µm, 8000 µm, 6000 µm, 4000 µm, or 2000 µm.

11. A diamond based electrochemical band sensor according to claim 9 or 10, wherein the length of each electrode is in a range 100 to 10000 µm, 200 to 8000 µm, 400 to 6000 µm, 600 to 4000 µm, 800 to 2000 µm, or 1000 to 2000 µm.

12. A diamond based electrochemical band sensor according to any preceding claim, wherein each boron doped diamond electrode has a surface area at the front sensing surface of at least 0.0001 mm², 0.001 mm², 0.005 mm², 0.010 mm², 0.015 mm², or 0.020 mm².

13. A diamond based electrochemical band sensor according to claim 12, wherein the surface area of each boron doped diamond electrode is equal to or less than 1.000 mm², 0.500 mm², 0.100 mm², 0.050 mm², or 0.030 mm².

14. A diamond based electrochemical band sensor according to any preceding claim, wherein a ratio of electrode spacing / electrode width is at least 1.5, 2, 4, 6, 8, 10, 50, 100, 500, or 1000.

15. A diamond based electrochemical band sensor according to claim 14, wherein the ratio of electrode spacing / electrode width is equal to or less than 10000, 5000, 1000, 500, 100, 50, 40, 30, 20 or 15.

16. A diamond based electrochemical band sensor according to claim 14 or 15, wherein the ratio of electrode spacing / electrode width is in a range 1.5 to 10000, 4 to 5000, 10 to 1000, 40 to 500, or 50 to 100.

17. A diamond based electrochemical band sensor according to any preceding claim, wherein each boron doped diamond band electrode has a width which varies by no more than ±20 µm, ±15 µm, ±10 µm, ±5 µm, ±2.5 µm, ±1 µm, ±0.1 µm, ±0.02 µm, ±0.005 µm, ±0.001 µm of a mean width along a length of the boron doped diamond band electrode at the front sensing surface.

18. A diamond based electrochemical band sensor according to any preceding claim, wherein at interfaces between the boron doped diamond band electrodes and non-conductive intrinsic layers of diamond, boron dopant varies by at least a factor of 3, 10, 30, 100, 300, 1000, 30000, or 100000 over a distance of no more than 10 µm, 3 µm, 1 µm, 0.3 µm, 0.1 µm, 0.03 µm, 0.01 µm, 0.003 µm, or 0.001 µm.

19. A diamond based electrochemical band sensor according to any preceding claim, wherein interfaces between the boron doped diamond band electrodes and non-conductive intrinsic layers of diamond are substantially free of impurities whereby, in a region either side of an interface extending to 20%, 50%, or 100% of a thickness of an associated boron doped diamond band electrode, an impurity concentration does not exceed 10¹⁴, 3 x 10¹⁴, 10¹⁵, 3 x 10¹⁵, 10¹⁶, 3 x 10¹⁶, or 10¹⁷, and does not vary in concentration by more than a factor of 2, 3, 5, 10, 30, 100, 300, or 1000.

20. A diamond based electrochemical band sensor according to any preceding claim, wherein at least a portion of each boron doped diamond band electrode comprises a boron concentration equal to or greater than 1 x 10²⁰ cm⁻³, 2 x 10²⁰ cm⁻³, 4 x 10²⁰ cm⁻³, 5 x 10²⁰ cm⁻³, 7 x 10²⁰ cm⁻³, 1 x 10²¹ cm⁻³, or 2 x 10²¹ cm⁻³.

## Patentansprüche

1. Elektrochemischer Bandsensor auf Diamantbasis, umfassend:
einen Diamantkörper (10) und
eine Vielzahl von bordotierten Diamantbandelektroden (12), die innerhalb des Diamantkörpers angeordnet sind, wobei mindestens ein Teil von jeder der Vielzahl von bordotierten Diamantbandelektroden mit Bor auf ein Niveau dotiert ist, das geeignet ist, um metallische Leitung zu erreichen, wobei die bordotierten Diamantelektroden durch nichtleitende intrinsische Schichten aus Diamant (14) beabstandet sind;
wobei der Diamantkörper (10) eine vordere Abfühlfläche (16) umfasst, wobei die Vielzahl von bordotierten Diamantbandelektroden (12) an der Abfühlfläche frei liegt und sich in länglicher Weise über die Fläche erstreckt,
und
**dadurch gekennzeichnet, dass**
jede bordotierte Diamantelektrode (12) ein Länge/Breite-Verhältnis von mindestens 10 an der vorderen Abfühlfläche (16) aufweist.

2. Elektrochemischer Bandsensor auf Diamantbasis nach Anspruch 1, bei dem der Diamantkörper eine Dicke und eine größte laterale Abmessung aufweist, wodurch ein Verhältnis von Dicke : größter lateraler Abmessung in einem Bereich von 1:10 bis 1,5:1, 1:5 bis 1:1 oder 1:3 bis 1:2 liegt.

3. Elektrochemischer Bandsensor auf Diamantbasis nach Anspruch 1 oder 2, bei dem jede bordotierte Diamantelektrode ein Länge/Breite-Verhältnis von mindestens 20, 30, 40, 50, 100, 500, 1000, 2000, 5000 oder 8000 an der vorderen Abfühlfläche aufweist.

4. Elektrochemischer Bandsensor auf Diamantbasis nach Anspruch 3, bei dem das Länge/Breite-Verhältnis nicht mehr als 15000, 10000, 8000, 5000, 2000 oder 1000 beträgt.

5. Elektrochemischer Bandsensor auf Diamantbasis nach Anspruch 3 oder 4, bei dem das Länge/Breite-Verhältnis in einem Bereich von 10 bis 15000, 20 bis 10000, 30 bis 5000 oder 50 bis 1000 liegt.

6. Elektrochemischer Bandsensor auf Diamantbasis nach einem der vorhergehenden Ansprüche, bei dem jede bordotierte Diamantelektrode eine Breite von mindestens 0,1 µm, 0,5 µm, 1 µm, 2 µm, 5 µm, 10 µm oder 15 µm aufweist.

7. Elektrochemischer Bandsensor auf Diamantbasis nach Anspruch 6, bei dem die Breite jeder Elektrode gleich oder kleiner als 100 µm, 80 µm, 60 µm, 40 µm, 20 µm, 10 µm, 1 µm oder 0,5 µm ist.

8. Elektrochemischer Bandsensor auf Diamantbasis nach Anspruch 6 oder 7, bei dem die Breite jeder Elektrode im Bereich von 0,1 bis 100 µm, 1 bis 80 µm, 5 bis 60 µm, 10 bis 40 µm oder 15 bis 30 µm liegt.

9. Elektrochemischer Bandsensor auf Diamantbasis nach einem der vorhergehenden Ansprüche, bei dem jede bordotierte Diamantelektrode eine Länge von mindestens 100 µm, 200 µm, 400 µm, 600 µm, 800 µm oder 1000 µm aufweist.

10. Elektrochemischer Bandsensor auf Diamantbasis nach Anspruch 9, bei dem die Länge jeder Elektrode gleich oder kleiner als 10000 µm, 8000 µm, 6000 µm, 4000 µm oder 2000 µm ist.

11. Elektrochemischer Bandsensor auf Diamantbasis nach Anspruch 9 oder 10, bei dem die Länge jeder Elektrode im Bereich von 100 bis 10000 µm, 200 bis 8000 µm, 400 bis 6000 µm, 600 bis 4000 µm, 800 bis 2000 µm oder 1000 bis 2000 µm liegt.

12. Elektrochemischer Bandsensor auf Diamantbasis nach einem der vorhergehenden Ansprüche, bei dem jede bordotierte Diamantelektrode eine Oberfläche an der vorderen Abfühlfläche von mindestens 0,0001 mm², 0,001 mm², 0,005 mm², 0,010 mm², 0,015 mm² oder 0,020 mm² aufweist.

13. Elektrochemischer Bandsensor nach Anspruch 12, bei dem die Oberfläche jeder bordotierten Diamantelektrode gleich oder kleiner als 1,000 mm², 0, 500 mm², 0,100 mm², 0,050 mm² oder 0,030 mm² ist.

14. Elektrochemischer Bandsensor auf Diamantbasis nach einem der vorhergehenden Ansprüche, bei dem ein Verhältnis von Elektrodenabstand / Elektrodenbreite mindestens 1,5, 2, 4, 6, 8, 10, 50, 100, 500 oder 1000 beträgt.

15. Elektrochemischer Bandsensor auf Diamantbasis nach Anspruch 14, bei dem das Verhältnis von Elektrodenabstand / Elektrodenbreite gleich oder kleiner als 10000, 5000, 1000, 500, 100, 50, 40, 30, 20 oder 15 ist.

16. Elektrochemischer Bandsensor auf Diamantbasis nach Anspruch 14 oder 15, bei dem das Verhältnis von Elektroden-abstand / Elektrodenbreite im Bereich von 1,5 bis 10000, 4 bis 5000, 10 bis 1000, 40 bis 500 oder 50 bis 100 beträgt.

17. Elektrochemischer Bandsensor auf Diamantbasis nach einem der vorhergehenden Ansprüche, bei dem jede bordotierte Diamantbandelektrode eine Breite aufweist, die um nicht mehr als ±20 µm, ±15 µm, ±10 µm, ±5 µm, ±2,5 µm, ±1 µm, ±0,1 µm, ±0,02 µm, ±0,005 µm, ±0,001 µm einer mittleren Breite entlang einer Länge der bordotierten Diamantbandelektrode an der vorderen Abfühlfläche variiert.

18. Elektrochemischer Bandsensor auf Diamantbasis nach einem der vorhergehenden Ansprüche, bei dem das Bordotiermittel an Grenzflächen zwischen den bordotierten Diamantbandelektroden und nichtleitenden intrinsischen Schichten aus Diamant um mindestens einen Faktor von 3, 10, 30, 100, 300, 1000, 30000 oder 100000 über eine Strecke von nicht mehr als 10 µm, 3 µm, 1 µm, 0,3 µm, 0,1 µm, 0,03 µm, 0,01 µm, 0,003 µm oder 0,001 µm variiert.

19. Elektrochemischer Bandsensor nach einem der vorhergehenden Ansprüche, bei dem Grenzflächen zwischen den bordotierten Diamantbandelektroden und nichtleitenden intrinsischen Schichten aus Diamant im Wesentlichen frei von Verunreinigungen sind, wobei eine Verunreinigungskonzentration in einer Region an jeder Seite einer Grenzfläche, die sich auf 20 %, 50 % oder 100 % einer Dicke einer dazugehörigen bordotierten Diamantbandenelektrode erstreckt, 10¹⁴, 3 x 10¹⁴, 10¹⁵, 3 x 10¹⁵, 10¹⁶, 3 x 10¹⁶ oder 10¹⁷ nicht überschreitet und in der Konzentration um nicht mehr als einen Faktor von 2, 3, 5, 10, 30, 100, 300 oder 1000 variiert.

20. Elektrochemischer Bandsensor auf Diamantbasis nach einem der vorhergehenden Ansprüche, bei dem mindestens ein Teil jeder bordotierten Diamantbandelektrode eine Borkonzentration gleich oder größer als 1 x 10²⁰ cm⁻³, 2 x 10²⁰ cm⁻³, 4 x 10²⁰ cm⁻³, 5 x 10²⁰ cm⁻³, 7 x 10²⁰ cm⁻³, 1 x 10²¹ cm⁻³ oder 2 x 10²¹ cm⁻³ umfasst.

## Revendications

1. Capteur électrochimique à bande à base de diamant, comportant :
un corps (10) en diamant ; et
une pluralité d'électrodes (12) à bande de diamant dopé au bore, disposées dans le corps en diamant,
dans lequel au moins une partie de chaque électrode parmi la pluralité d'électrodes à bande de diamant dopé au bore est dopée au bore à un niveau approprié pour obtenir une conduction métallique, les électrodes à diamant dopé au bore étant espacées les unes des autres par des couches intrinsèques non conductrices de diamant (14),
dans lequel le corps (10) en diamant comporte une surface frontale détectrice (16), la pluralité d'électrodes (12) à bande de diamant dopé au bore étant exposées à ladite surface détectrice et s'étendant d'une manière allongée d'un côté à l'autre de ladite surface,
et
**caractérisé en ce que** chaque électrode (12) à diamant dopé au bore a un rapport longueur/largeur d'au moins 10 sur la surface frontale détectrice (16).

2. Capteur électrochimique à bande à base de diamant selon la revendication 1, dans lequel le corps en diamant a une épaisseur et une dimension latérale la plus grande, telles qu'un rapport épaisseur/dimension latérale la plus grande est dans une plage de 1/10 à 1, 5/1, de 1/5 à 1/1 ou de 1/3 à 1/2.

3. Capteur électrochimique à bande à base de diamant selon la revendication 1 ou 2, dans lequel chaque électrode à diamant dopé au bore a un rapport longueur/largeur d'au moins 20, 30, 40, 50, 100, 500, 1000, 2000, 5000 ou 8000 sur la surface frontale détectrice.

4. Capteur électrochimique à bande à base de diamant selon la revendication 3, dans lequel le rapport longueur/largeur n'est pas supérieur à 15 000, 10 000, 8000, 5000, 2000 ou 1000.

5. Capteur électrochimique à bande à base de diamant selon la revendication 3 ou 4, dans lequel le rapport longueur/largeur est dans une plage de 10 à 15 000, de 20 à 10 000, de 30 à 5000 ou de 50 à 1000.

6. Capteur électrochimique à bande à base de diamant selon l'une quelconque des revendications précédentes, dans lequel chaque électrode à diamant dopé au bore a une largeur d'au moins 0,1 µm, 0,5 µm, 1 µm, 2 µm, 5 µm, 10 µm ou 15 µm.

7. Capteur électrochimique à bande à base de diamant selon la revendication 6, dans lequel la largeur de chaque électrode est égale ou inférieure à 100 µm, 80 µm, 60 µm, 40 µm, 20 µm, 10 µm, 1 µm ou 0,5 µm.

8. Capteur électrochimique à bande à base de diamant selon la revendication 6 ou 7, dans lequel la largeur de chaque électrode est dans une plage de 0,1 à 100 µm, de 1 à 80 µm, de 5 à 60 µm, de 10 à 40 µm ou de 15 à 30 µm.

9. Capteur électrochimique à bande à base de diamant selon l'une quelconque des revendications précédentes, dans lequel chaque électrode à diamant dopé au bore a une longueur d'au moins 100 µm, 200 µm, 400 µm, 600 µm, 800 µm ou 1000 µm.

10. Capteur électrochimique à bande à base de diamant selon la revendication 9, dans lequel la longueur de chaque électrode est égale ou inférieure à 10 000 µm, 8000 µm, 6000 µm, 4000 µm, ou 2000 µm.

11. Capteur électrochimique à bande à base de diamant selon la revendication 9 ou 10, dans lequel la longueur de chaque électrode est dans une plage de 100 à 10000 µm, de 200 à 8000 µm, de 400 à 6000 µm, de 600 à 4000 µm, de 800 à 2000 µm ou de 1000 à 2000 µm.

12. Capteur électrochimique à bande à base de diamant selon l'une quelconque des revendications précédentes, dans lequel chaque électrode à diamant dopé au bore a, sur la surface frontale détectrice, une superficie d'au moins 0,0001 mm², à 0,001 mm², 0,005 mm², 0,010 mm², 0,015 mm² ou 0,020 mm².

13. Capteur électrochimique à bande à base de diamant selon la revendication 12, dans lequel la superficie de chaque électrode à diamant dopé au bore est égale ou inférieure à 1,000 mm², 0,500 mm², 0,100 mm², 0,050 mm² ou 0,030 mm².

14. Capteur électrochimique à bande à base de diamant selon l'une quelconque des revendications précédentes, dans lequel un rapport d'espacement d'électrode/largeur d'électrode est d'au moins 1,5, 2, 4, 6, 8, 10, 50, 100, 500 ou 1000.

15. Capteur électrochimique à bande à base de diamant selon la revendication 14, dans lequel le rapport d'espacement d'électrode/largeur d'électrode est égal ou inférieur à 10 000, 5000, 1000, 500, 100, 50, 40, 30, 20 ou 15.

16. Capteur électrochimique à bande à base de diamant selon la revendication 14 ou 15, dans lequel le rapport d'espacement d'électrode/largeur d'électrode est dans une plage de 1,5 à 10 000, de 4 à 5000, de 10 à 1000, de 40 à 500 ou de 50 à 100.

17. Capteur électrochimique à bande à base de diamant selon l'une quelconque des revendications précédentes, dans lequel chaque électrode à bande en diamant dopé au bore a une largeur qui varie d'un maximum de ± 20 µm, ± 15 µm, ± 10 µm, ± 5 µm, ± 2,5 µm, ± 1 µm, ± 0,1 µm, ± 0,02 µm, ± 0,005 µm, ± 0,001 µm d'une largeur moyenne le long d'une longueur de l'électrode à bande en diamant dopé au bore sur la surface frontale détectrice.

18. Capteur électrochimique à bande à base de diamant selon l'une quelconque des revendications précédentes, dans lequel, à des interfaces entre les électrodes à bande de diamant dopé au bore et les couches intrinsèques non conductrices de diamant, le dopage au bore varie d'au moins un facteur de 3, 10, 30, 100, 300, 1000, 30 000 ou 100 000 sur une distance non supérieure à 10 µm, 3 µm, 1 µm, 0,3 µm, 0,1 µm, 0,03 µm, 0,01 µm, 0,003 µm ou 0,001 µm.

19. Capteur électrochimique à bande à base de diamant selon l'une quelconque des revendications précédentes, dans lequel des interfaces entre les électrodes à bande de diamant dopé au bore et des couches intrinsèques non conductrices de diamant sont sensiblement exemptes d'impuretés, dans lequel, dans une région de part et d'autre d'une interface s'étendant jusqu'à 20 %, 50 % ou 100 % d'une épaisseur d'une électrode associée à bande de diamant dopé au bore, une concentration d'impuretés ne dépasse pas 10¹⁴, 3 x 10¹⁴, 10¹⁵, 3 x 10¹⁵, 10¹⁶, 3 x 10¹⁶ ou 10¹⁷ et son facteur de concentration ne varie pas de plus de 2, 3, 5, 10, 30, 100, 300 ou 1000.

20. Capteur électrochimique à bande à base de diamant selon l'une quelconque des revendications précédentes, dans lequel au moins une partie de chaque électrode à bande de diamant dopé au bore comporte une concentration de bore égale ou supérieure à 1 x 10²⁰ cm⁻³, 2 x 10²⁰ cm⁻³, 4 x 10²⁰ cm⁻³, 5 x 10²⁰ cm⁻³, 7 x 10²⁰ cm⁻³, 1 x 10²¹ cm⁻³ ou 2 x 10²¹ cm⁻³.
